# EUROPEAN PATENT SPECIFICATION

(11) **EP 3 325 077 B1**
(45) Date of publication and mention of the grant of the patent: **12.08.2026**
(21) Application number: 16828633.4
(22) Date of filing: 22.07.2016
(51) Int. Cl.: A61M 25/092, A61M 25/095, A61M 25/098, A61M 25/14

(54) **GUIDEWIRE NAVIGATION SYSTEM WITH DIRECT VISUALIZATION FEATURE**
FÜHRUNGSDRAHTNAVIGATIONSSYSTEM MIT DIREKTER VISUALISIERUNGSFUNKTION
SYSTÈME DE NAVIGATION DE FIL-GUIDE À FONCTION DE VISUALISATION DIRECTE

(30) Priority: 22.07.2015 US 201562195662 P
(43) Date of publication of application: 30.05.2018
(73) Proprietor: Cardioguidance Biomedical, LLC, Arlington, VA 22207 (US)
(72) Inventor: MILLER, Scott, Arlington, VA 22207 (US)
(74) Representative: Maschio & Soames IP Ltd
(86) International application number: PCT/US2016/043652
(87) International publication number: WO 2017/015594

(56) References cited:
- WO-A1-2014/155794
- US-A- 5 643 218
- US-A- 5 643 218
- US-A- 6 004 269
- US-A- 6 004 269
- US-A1- 2008 172 037
- US-A1- 2012 041 534
- US-A1- 2012 253 193
- US-A1- 2012 253 193
- US-A1- 2014 135 576
- US-A1- 2016 045 101

## Description

### FIELD

The present disclosure relates to a medical device, and more particularly to a guidewire navigation system having an incorporated visualization feature to enable the user to both guide medical instruments toward, and also visualize while navigating to, remote spaces.

### BACKGROUND

Guidewires are currently being used in medical procedures to guide catheters, cannulas, sheaths, or other devices from a remote site to a treatment site within a patient. These treatment sites can sometimes be hard to reach, narrow, confined areas, and the pathway tortuous. In a typical procedure, a guidewire is introduced from a remote part of the body through the patient's lumen and toward the treatment site until it encounters an obstruction. When this happens, an exchange catheter is advanced over the wire, the wire is withdrawn, and a new wire with different (usually larger) dimensions is advanced to the end of the exchange catheter. Then, the catheter is withdrawn, and the new wire is advanced further until it cannot advance any more, or until a different dimensioned wire is needed. At this point, the exchange cycle repeats itself, with differently dimensioned wires being swapped out using an exchange catheter until the target is reached. This is all performed under fluoroscopic guidance typically, and is time consuming and requires the physician to rely on two dimensional imaging for his or her decision making. Document WO 2014/155794 A1 discloses a catheter system with variable stiffness and a camera.

Accordingly, it is desirable to provide a guidewire navigation system having visualization features incorporated into the system itself, so that the wire and catheter exchange cycle can be performed without the need for additional fluoroscopic guidance or any further visualization guidance, unless such additional guidance is desired.

### SUMMARY

The invention is defined by claim 1. The present disclosure provides a navigation system with a direct visualization feature. The system may comprise multiple, slidable members of different size and flexibility. The system can be advanced and retracted to change the overall diameter, flexibility and stiffness of the system. Also included is a lock to fix the members of the system in relative position to one another. These attributes of the system allow one to navigate to the target site without having to engage in multiple guidewire exchanges, and without having to rely on a single imaging modality.

In some embodiments, a navigation system with a remote visualization element for navigating in remote spaces in medical and non-medical applications is provided. The system may include multiple members to allow navigation without having to change between guidewires, catheters and other navigation instruments to access the desired navigation point. The system eliminates the need to exchange between multiple devices to successfully navigate to the desired navigation point, while also providing a system with therapeutic capability to further diagnose and treat disease.

The system may include an inner member which includes a remote visualization element. This visualization element connects to a processor and light source for imaging tissue or other matter at a remote distance from the user. The navigation system may also include one or more outer members, which may be coaxial or otherwise. The outer member(s) are configured for navigating in a lumen, and for increasing the overall diameter and stiffness of the system, as needed at any point in time during the procedure, without an exchange between instruments, such as to round turns, navigate bifurcations, and to cross tortuous anatomy. The system is also configured so that its stiffness can be adjusted to support the advancement of larger devices over the navigation system.

The visualization feature incorporated into the present system allows the system to function as a "seeing" navigation system with the ability to independently navigate by direct visualization through tortuous anatomy, lumens and other hard to reach locations, while also having the ability to change the system's overall diameter, stiffness and flexibility, as well as to infuse infusible matter. The system also contains radiopaque materials, so navigation can take place through other means, including a combination of fluoroscopic guidance, ultrasound guidance, electromagnetic guidance, and direct visualization, as needed, improving one's ability to navigate through a variety of environments. This feature provides an advantage over the current state of the art, which typically involves navigation with a single imaging modality, such as fluoroscopy, and a single navigation capability, such as navigation with one medical guidewire at a time and then engaging in exchanges between multiple guidewire to obtain different diameters and stiffness levels. The present system provides a way to navigate with multiple imaging modalities and with multiple navigation features, including the ability to change size, diameter and stiffness on the fly, without needing to use a catheter to exchange one wire for another in the time consuming practice that is the conventional practice today.

The system may be used in medical applications, including, among others, gastrointestinal, vascular, coronary, urological, gynecological, pulmonary, ear nose and throat, neuro, orthopedic and lesser or minimally invasive surgery applications. The system may also be used in non-medical applications where navigation and visualization remotely is beneficial, including, among others, the inspection of gas lines, pipelines, hydraulic lines, seeing remotely around corners and underneath openings, among others.

In an exemplary embodiment, the inner member with the remote visualization element has a diameter of less than 0.356 mm (0.014 inches) and the system has an overall diameter of 0.889 mm (0.035 inches) or less. The size of the various members may be larger or smaller in other embodiments, including the overall diameter of the system.

In another exemplary embodiment, the visualization element may be in one or more of the outer elements of the system.

The visualization element can include the ability to change the spectrum of light in order to enhance the ability of the system to inspect, diagnose and treat matter. In some embodiments, the visualization element may be able to engage in narrow band imaging, high definition imaging, infrared imaging, ultrasound, and other forms of imaging and light spectrum to improve the information provided to the user through the visualization element.

The system can include one or more locking mechanisms to affix the members together so the system can be navigated and used as a larger diameter guidewire with remote visualization, as well as be used unlocked so the individual members can be moved forward and back, as needed, to advance the system.

In some embodiments, a third member may be part of the system to act as a stiffener between the first and second members, and the third member may also act as a lock to affix the members together to navigate as a single system with adjustable flexibility and stiffness. In other embodiments, the system may contain a lock as a fourth element of the system.

The members may be made of biocompatible materials, including for example, metals such as steel, nitinol, cobalt-chromium and other materials, such as polyetheretherketone (PEEK), polymers, and other elastic materials. The members may be rigid or flexible, spiral cut in part or in total, covered with coatings in whole or in part. The coatings, in some embodiments, may include polytetrafluoroethylene (PTFE), polyethylene terepthalate (PET) and other biocompatible materials. The coatings may also include coatings that change the performance of the material in fluid, including hydrophobic, superhydrophobic, and hydrophilic coatings, and coatings that are anti-inflammatory, anti-bacterial, anti-microbial, and coatings that are electrically responsive.

One or more of the members may be steerable. This may be achieved by having an outer member with steerable capabilities to turn and guide the system, or by having a steerable inner member, which the outer members then advance over to adjust flexibility, stiffness and diameter. In addition, through the use of the locking mechanism, the members of the system can be locked together so the system has distinct attributes associated with the combined /locked features of the system, including steerability, flexibility, diameter, imaging, and visualization ability in a unique manner based on the combined features created by locking or fixing the relative properties of the members of the system.

In some embodiments, the steerability of the system may be achieved through the transmission of torque through the system, by twisting one or more members at the proximal end and having the torque transfer to the system to cause the distal end of one or more members to turn. The system may also be steerable by having an outer member or an inner member, or a combination of members with cables, piezo electric material, electrically activated polymers, interlocking members or other elements to transmit force selectively across a portion or across the entire system to cause it to turn one or more directions. The system may also be steerable by having small robotic elements, including ultrasound driven or electric motors and/or organic conductors, with the ability to change shape as intended to steer one or more members of the device. The device may be navigated and articulated by the user directly, or as part of an automated or robotic system in which the user's input is translated through the system through various means, including for example, cables, power connectors, motors, electromagnetic energy, slideable sheaths, haptics, computer-guided and directed input, and other means to direct and guide the device to its intended location, including to specific diagnosis and treatment objectives in a patient, or in non-medical applications, to a desired remote location.

The inner member may also have variations that may or may not include visualization. The inner member may have a cutting knife that is fixed or can be advanced and withdrawn to cut, excise or dissect tissue, plaque or other matter. In some embodiments, the knife may be straight, curved, a corkscrew or other shape for excising, cutting or dissecting tissue or other matter.

One of the members may have a grasping element to biopsy tissue, or one of members may include a brush to capture cells or other matter. The inner member may also be a solid element, and one or more of the members may have the ability to be energized to affect tissue and other matter, including blood clots. The energy may be directed at specific tissue or other matter, such as blood clots or a cancerous tumor, including creating an electromagnetic field focused on a specific treatment or diagnostic area to facilitate the guidance of instruments or drugs responsive to energy to a target location.

One or more of the members may have a pressure sensor or temperature sensor to provide the user with additional feedback of the area in which the system is navigating towards, diagnosing and treating. One or more sensors may be arrayed to create three dimensional or four dimensional imaging.

The system may include infusion management capabilities to infuse various fluids, gases, imaging agents and other infusible matter through the system. This includes the infusion of contrast media, carbon dioxide, oxygen, water, pharmaceuticals, glues, and other infusible matters. The infusion system includes a valve to prevent the influx of air or other unwanted matter and to prevent backflow once the matter is infused in to the system. The infusion system may include a flushing port to remove matter from the system so additional infusible matter can be infused without mixing multiple types of infusible matter. The infusion management system may include one or more ports with locking elements to connect to bags, syringes or other holders and dispensers of infusible matter. The infusion management system may also include a mixing element to combine infusible matter prior to infusion, or to infuse multiple matters simultaneously. If the infusible matter has a toxic element, such as chemotherapy agents or radioactive isotopes (such as radiospheres for treatment of liver cancer), the infusion management system may include shielding material to protect healthcare workers, including shielding material composed of lead and other protective materials.

The infusion system may connect to the other members through a connector in alignment with the axis of the system, or with a connector that is at an angle to the axis of the system. The ability to infuse infusible matter into the system may also be integrated into a member of the system, including as part of a locking mechanism that can also infuse matter, or a member of the system that can change the diameter, stiffness and flexibility of the system and infuse matter.

The infusion system may be detachable from the guidewire navigation system, or all or a portion of the infusion system may an attached or integrated part of the system.

The infusion management system may include the ability to infuse matter systematically over a defined period of time. An example of this is infusion beads which have been mixed by the infusion management system with a chemotherapy agent and contrast media and which are then infused through the system to a liver tumor over a set infusion time to insure proper dispersion at the location of the tumor. In some embodiments, the timed delivery may involve an infusion element that connects to a port that is part of the infusion management system. In some embodiments, the timed delivery may take place through an electric motor that creates pressure to advance the infusible matter in a sequential fashion or other manner, a spring that mechanically is released over time to apply pressure to a plunger or other element to infuse the matter, a gravity-fed system in which the infusible matter is fed by gravity with timed release of the infusible matter from the gravity-fed system, or a pump based system that pumps the infusible manner into the system in a manner programmed or determined by the user.

In some embodiments, the fluid management system may be composed of a hemostatic valve and one or more luer locks and stopcocks, with a Y-shaped connector, to manage and infuse the flow of fluid, drugs, beads (including micro and nano particles) and other matter through the lumens of the navigation system.

The members of the system may include a biopsy instrument which can be used to sample one or more locations of tissue or other matter. In some embodiments, the biopsy element may be an inner member or other member of the system, or it may be contained in a sheath that is pulled back to excise tissue or exposed as one of the outer members of the system. The biopsy instrument may have the ability to suction excised tissue through the biopsy element to a catch element outside of the system, or the biopsy element may have an internal element to capture biopsied tissue, to enable multiple biopsies while inside a patient. In some embodiments, the tissue contacting aspects of the biopsy element may involve jaws, articulating jaws, a coring element (which may excise tissue directly in front of the element through penetration, by penetrating and rotating the device to excise the tissue), or with a sweeping or articulating arm element to excise tissue.

In some embodiments, the system may include a brush as part of a member to advance to capture cells for diagnosis and testing.

In some embodiments, the system may include a member with the ability to capture stones or other matter in a basket or other capturing element. In some embodiments, the basket may have the ability to articulate, to close and to open, including the ability to advance over a stone and grasp or capture the stone, using the visualization capability of the system to position the basket precisely. In still other embodiments, the system may include a laser element to ablate the stones prior to removal.

In some embodiments, the system may include a member that can articulate and cut tissue, including enlarging the access of a biliary duct or similar anatomical structure. This cutting may occur through mechanical means, such as with a cutting member that includes a sharpened element for cutting tissue. Alternatively, the cutting may be achieved through different means, such as with the delivery of an energy modality, including with a blade, wire or member edge, to cut tissue, or the cutting can be achieved through a combination of these elements. The energy modalities may include monopolar energy, biopolar energy, microwave energy, and forms of ultrasound energy.

In accordance with the invention, a navigation system is provided having an inner member and an outer member having an inner diameter greater than the outer diameter of the inner member. The outer member comprises a flexible tube with different regions of flexibility. The system further includes a lumen between the inner and outer members and an alternative lumen through the outer member. One of the members may have an imaging capability comprising ultrasound, magnetic imaging, a remote visualization element, or other visualization or imaging modality in addition to fluoroscopic imaging. In some embodiments, one of the members includes a cutting wire. In other embodiments, one of the members includes a needle knife for dissecting and energizing tissue. In still other embodiments, the system is remotely steerable through one or more of its members.

It is to be understood that both the foregoing general description and the following detailed description are exemplary and explanatory only and are not restrictive of the disclosure. Additional features of the disclosure will be set forth in part in the description which follows or may be learned by practice of the disclosure.

### BRIEF DESCRIPTION OF THE DRAWINGS

The accompanying drawings, which are incorporated in and constitute a part of this specification, illustrate several embodiments of the disclosure and together with the description, serve to explain the principles of the disclosure.
FIG. 1 shows a perspective view of an exemplary embodiment of a guidewire system of the present disclosure.
FIG. 2 shows a perspective view of another exemplary embodiment of a guidewire system of the present disclosure.
FIG. 3 shows a perspective view of still another exemplary embodiment of a guidewire system of the present disclosure.
FIG. 4 shows a perspective view of yet another exemplary embodiment of a guidewire system of the present disclosure.
FIG. 5 shows an exploded perspective view of an exemplary embodiment of a guidewire system of the present disclosure.
FIG. 6 shows an enlarged perspective view of an exemplary embodiment of an outer member of the present disclosure.
FIG. 7 shows an exploded perspective view of another exemplary embodiment of a guidewire system of the present disclosure.
FIG. 8 shows an exploded perspective view of still another exemplary embodiment of a guidewire system of the present disclosure.
FIG. 9 shows a perspective view of yet another exemplary embodiment of a guidewire system of the present disclosure.
FIG. 10 shows a perspective view of still another exemplary embodiment of a guidewire system of the present disclosure.
FIG. 11 shows a perspective view of further still another exemplary embodiment of a guidewire system of the present disclosure.

### DETAILED DESCRIPTION

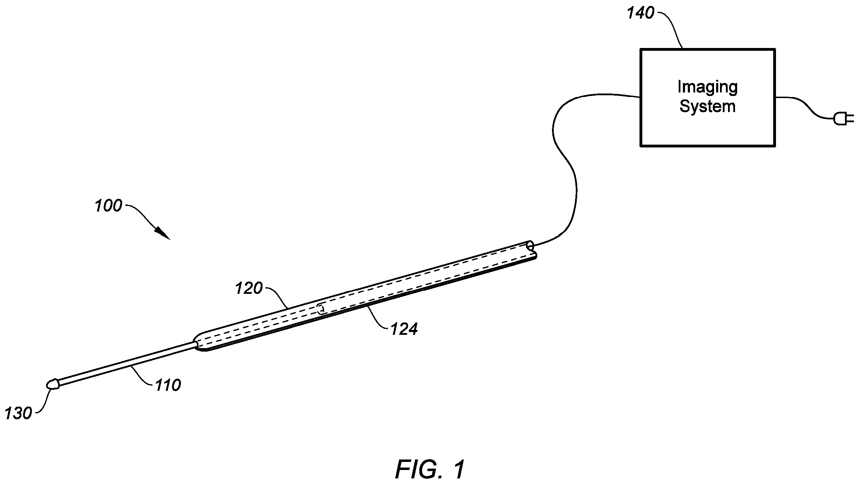

Turning now to the drawings, FIG. 1 shows an exemplary embodiment of a guidewire system of the present disclosure. The system 100 may comprise an inner member 110 and an outer member 120. The inner member 110 may have incorporated therein a visualization element 130 which connects to an imaging system 140. This enables direct visualization and imaging during navigation.

The outer member 120 may further include a coating 124. As described, the coating 124 may comprise PTFE, PET, or other biocompatible materials. The coating 124 may also change the performance of the material in fluid, such as by being a hydrophobic, superhydrophobic, or hydrophilic coating. The coating 124 may also be anti-inflammatory, anti-bacterial, anti-microbial, and/or electrically responsive.

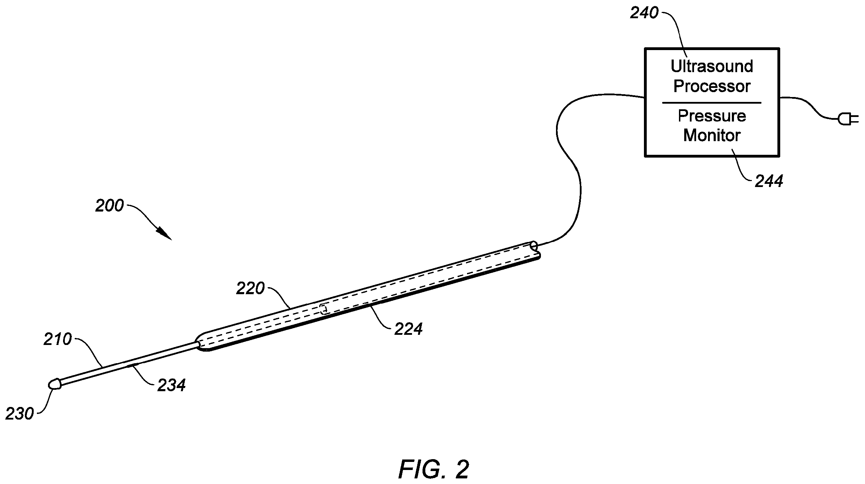

FIG. 2 shows another exemplary embodiment of a guidewire system of the present disclosure. The system 200 may comprise an inner member 210 and outer member 220. The inner member 210 may have incorporated therein an ultrasound sensor 230 and/or a pressure sensor 234, or multiple sensors, which then connects to an ultrasound processor 240 and/or pressure monitor 244. Similar to the system 100 of FIG. 1, the outer member 220 may also comprise a coating 224. The coating may comprise PTFE, PET, or other biocompatible materials. In one exemplary embodiment, both the inner and outer members 210, 220 may include a coating. The materials of the coatings may be the same, or different. For example, the inner member 210 may have a PTFE coating, while the outer member may have a PET coating, or vice versa.

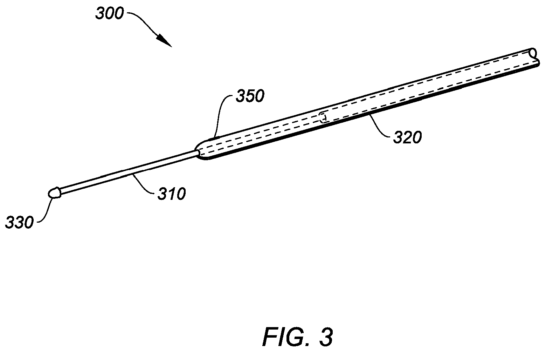

FIG. 3 shows still another exemplary embodiment of a guidewire system of the present disclosure. The system 300 may comprise an inner member 310 and outer member 320. The inner member 310 may have incorporated therein a cutting or dissecting element 330, which can be connected to an external power supply source. To facilitate the process, the outer member 320 may have incorporated therein a camera or visualization feature 350. Either the inner member 310 or the outer member 320 may have a coating as previously described.

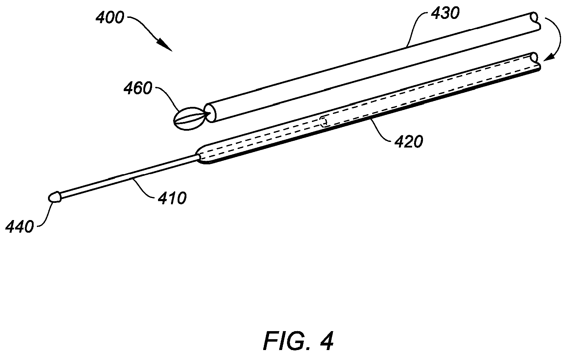

FIG. 4 shows yet another exemplary embodiment of a guidewire system of the present disclosure. The system 400 may comprise an inner member 410, intermediate member 420, and outer member 430. Outer member 430 may have a coating as described for outer member 110 of the system 100 of FIG. 1. The inner member 410 may have incorporated therein a visualization element 440, which then connects to an imaging system (not shown), similar to the system 100 described in FIG. 1. However, in addition, the system 400 may comprise an outer member 430 having a retrieval element 460. This retrieval element 460 may comprise a basket, as shown, for tissue and biomatter collection. The basket may be configured to be collapsible and expandable, if so desired.

One contemplated use for system 400 may be to capture stones (e.g., kidney stones) or other matter in the basket 460. In some embodiments, the basket 460 may have the ability to articulate, to close and to open, including the ability to advance over a stone and grasp or capture the stone, using the visualization element 440 of the system 400 to position the basket 400 precisely. In addition, a laser element (not shown) may be incorporated into the system 400 so that ablation of the stone(s) may be achieved prior to capturing and removing them from the patient. The laser element may be incorporated into the inner member 410, or another member separate from that shown.

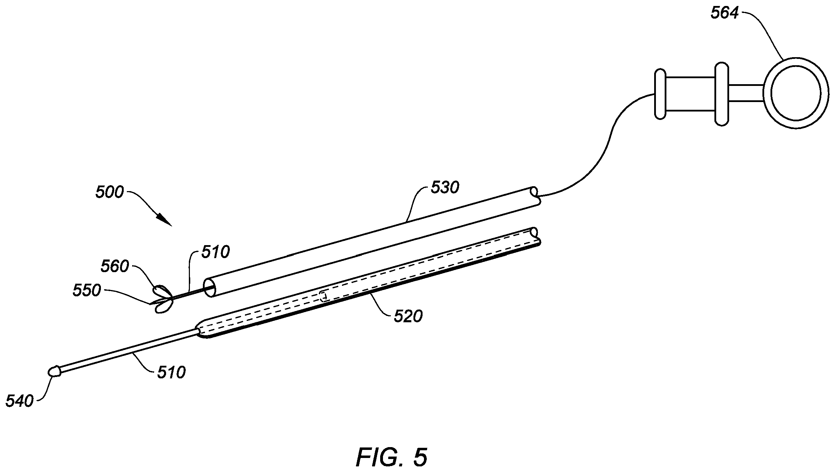

FIG. 5 shows even still another exemplary embodiment of a guidewire system of the present disclosure. The system 500 may comprise an inner member 510, intermediate member 520 and outer member 530. Outer member 530 may have a coating as described for outer member 110 of the system 100 of FIG. 1. The inner member 510 may have incorporated therein a visualization element 540, which then connects to an imaging system (not shown), similar to the system 100 described in FIG. 1.

In addition, the system 500 may comprise tissue cutting or excision and removal features. For example, as shown, the inner and outer members 510, 530 may be provided at their distal ends 512, 532 with a cutting element such as a knife 550 and biopsy forceps 560. As previously described, the inner member 510 may have a cutting knife 550 that is fixed or can be advanced and withdrawn to cut, excise or dissect tissue, plaque or other matter. In some embodiments, the knife may be straight, curved, a corkscrew or other shape for excising, cutting or dissecting tissue or other matter. The outer member 530 may have a biopsy forceps 560 at its distal end 532 to remove the excised tissue, and be in communication with a biopsy forceps controller 564, as shown.

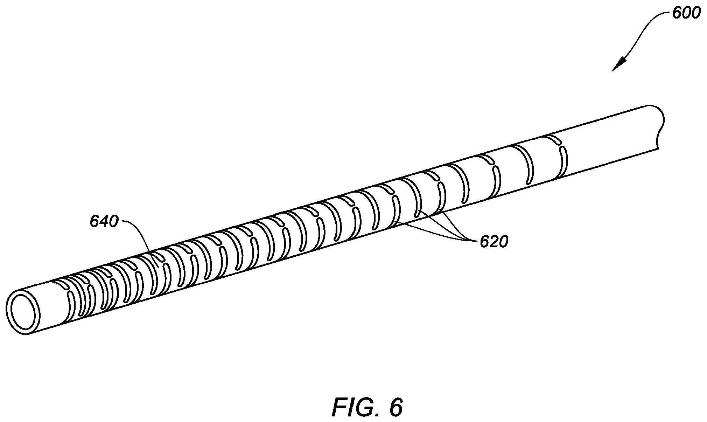

FIG. 6 shows an exemplary embodiment of an outer member 600 that can be used in any of the systems of the present disclosure. The outer member 600 may also be used as an intermediate member in some applications. As shown, the outer member 600 may include a series of tapered laser cuts 620 that enable selective regions of the member 600 to have a different flexibility than an adjacent region. This feature helps steerability and navigation of the system as a whole.

Additionally, the outer member 600 may further include a coating 640. As described, the coating 640 may comprise PTFE, PET, or other biocompatible materials. The coating 640 may also change the performance of the material in fluid, such as by being a hydrophobic, superhydrophobic, or hydrophilic coating. The coating 640 may also be anti-inflammatory, anti-bacterial, anti-microbial, and/or electrically responsive, such as by comprising an electrically activated polymer.

As mentioned above, the systems of the present disclosure can include one or more locking mechanisms to affix the members together so the system can be navigated and used as a larger diameter guidewire with remote visualization, as well as be used unlocked so the individual members can be moved forward and back, as needed, to advance the system. Through the use of the locking mechanism, the members of the system can be locked together so the system has distinct attributes associated with the combined /locked features of the system, including steerability, flexibility, diameter and visualization ability in a unique manner based on the combined features created by locking or fixing the relative properties of the members of the system.

Furthermore, as also mentioned above, the system may include infusion management capabilities to infuse various fluids, gases, imaging agents and other infusible matter through the system. This includes the infusion of contrast media, carbon dioxide, oxygen, water, pharmaceuticals, glues, and other infusible matters. The infusion system includes a valve to prevent the influx of air or other unwanted matter and to prevent backflow once the matter is infused in to the system. The infusion system may include a flushing port to remove matter from the system so additional infusible matter can be infused without mixing multiple types of infusible matter. The infusion management system may include one or more ports with locking elements to connect to bags, syringes or other holders and dispensers of infusible matter. The infusion management system may also include a mixing element to combine infusible matter prior to infusion, or to infuse multiple matters simultaneously. If the infusible matter has a toxic element, such as chemotherapy agents or radioactive isotopes (such as radiospheres for treatment of liver cancer), the infusion management system may include shielding material to protect healthcare workers, including shielding material composed of lead and other protective materials.

The infusion system may connect to the other members through a connector in alignment with the axis of the system, or with a connector that is at an angle to the axis of the system. The ability to infuse infusible matter into the system may also be integrated into a member of the system, including as part of a locking mechanism that can also infuse matter, or a member of the system that can change the diameter, stiffness and flexibility of the system and infuse matter. The infusion system may be detachable from the system, or all or a portion of the infusion system may an attached or integrated part of the navigation system.

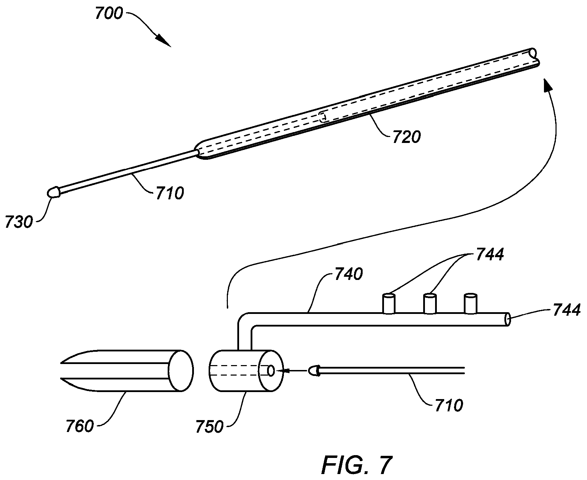

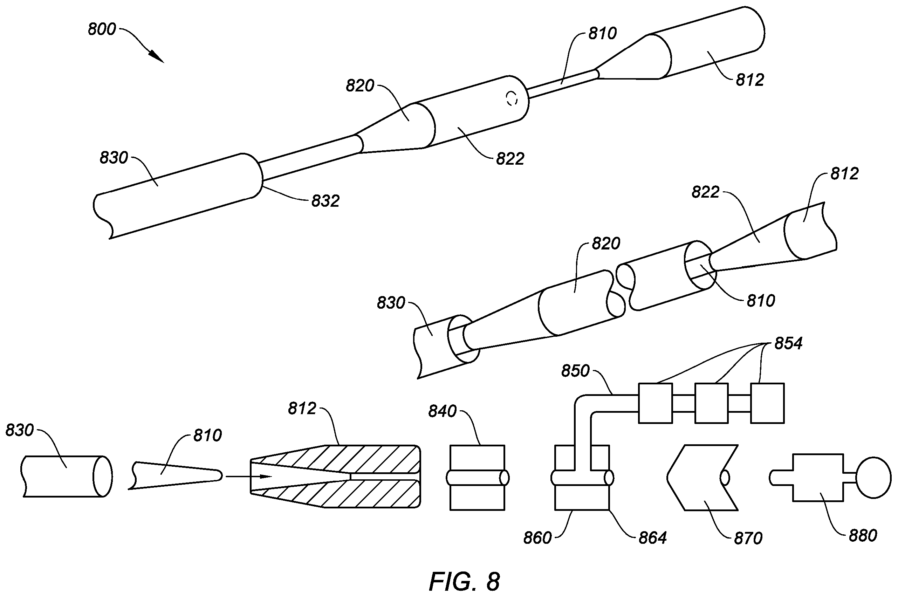

FIGS. 7 and 8 illustrate these principles. As FIG. 7 shows, the system 700 may comprise an inner member 710 and outer member 720. The inner member 710 may have incorporated therein a visualization element 730 which connects to an imaging system (not shown) to enable direct visualization during navigation.

As further shown, the system 700 may include a connector 740 such as for example a Y-connector having one or more injection ports 744. The Y-connector can comprise a valve 750 at one end, such as a hemostatic valve 750. This valve 750 can be configured as part of the lock or it can be configured to connect to the inner member 710 with a locking member 760. The other end of the Y-connector can be attached to a fluid source for infusion of the infusible material.

FIG. 8 shows an exemplary embodiment of a guidewire system 800 in which the inner member 810, intermediate member 820, and outer member 830 may comprise locking elements 812, 822, 832 in order to lock together and work in unison as a single member. As in the system 700 of FIG. 7, the system 800 may incorporate infusion features. For instance, the locking member 812 may also connect the inner member 810 to other components, such as a valve 840 that attaches to a connector 850 with one or more ports 854 for the infusion of flowable material. The connector 850 may be in fluid communication with other components, such as a mixer 860, a timed infusion element 870, and a flushing element 880. In cases where the material to be infused has a toxic element to it, the mixer 860 may having shielding 864 to protect the user.

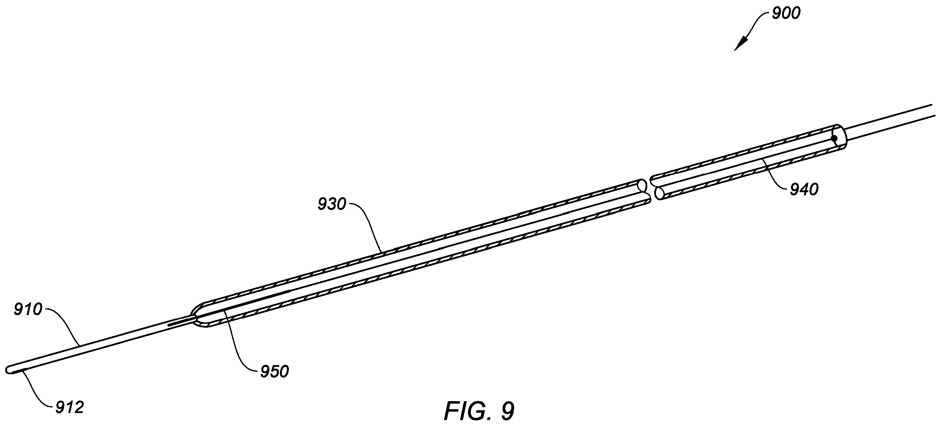

FIG. 9 shows another exemplary embodiment of a guidewire system system 900 in which the system 900 includes an imaging element 912, which could be, for example, a direct visualization element, an ultrasound element, or other visualization or imaging element. In one embodiment, the imaging element 912 is associated with the inner member 910. The guidewire system 900 may have an outer element 940 that is steerable and, in one embodiment, is associated with the outer member 930. In some embodiments, the system 900 can further include a cutting element 950, such as a bipolar or monopolar wire, for cutting tissue or other material. This cutting element 950 may be associated with a portion of the outer element 940, as shown.

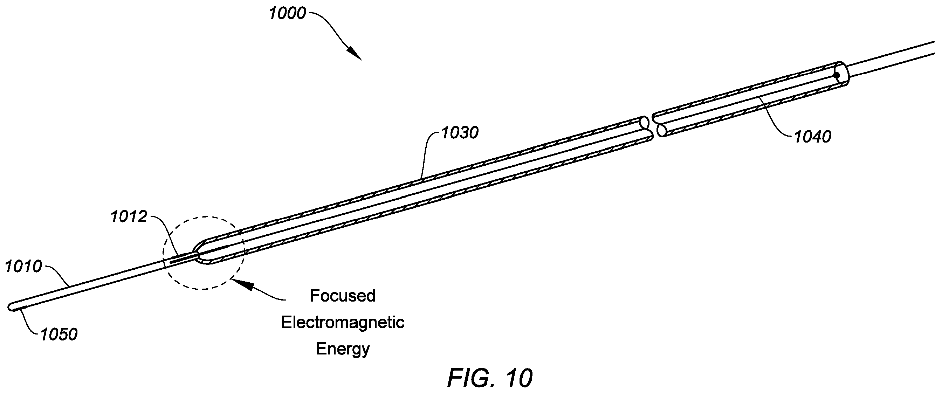

FIG. 10 shows still another exemplary embodiment of a guidewire system 1000 in which the system 1000 may include a cutting element 1050, such as for instance an energizable needle knife, for cutting tissue. In some embodiments, the cutting element 1050 may be associated with the inner member 1010. Additionally, the system 1000 may have an imaging element 1012, which could be a direct visualization element, an ultrasound element, or other visualization or imaging element. This imaging element 1012 may be associated with the outer member 1030. The outer member 1030 may also have an associated steerable element 1040 for steering the system 1000 and the ability to deliver focused electromechanical energy to guide the delivery of the needle knife 1050 or other instrument. It is contemplated that the needle knife 1050 may be configured to cut mechanically, or by using energy such as a bipolar or monopolar energy source, for cutting tissue or other material. One or both of the inner and outer members may further include a coating, as previously described.

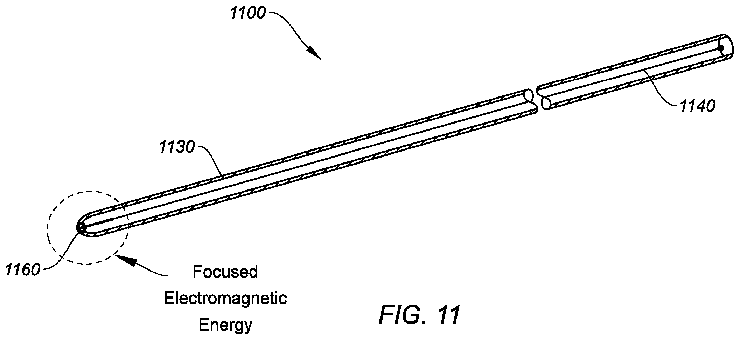

FIG. 11 shows further still another exemplary embodiment of a guidewire system 1100 in which the inner member has been withdrawn (not shown) and an outer member 1130 is configured for infusion. For example, as shown, the outer member 1130 can have an inner lumen 1160 for infusion of material, such as delivery of a drug. The outer member 1130 can also include a focused electromechanical energy element to focus electromechanical energy on a target, such as a blood clot or a tumor, to guide the delivery of an infused medium or matter, such as a drug with a magnetizable nanoparticle, to a blood clot, tumor or other target. Similar to the previous systems, the outer member 1130 may also have an associated steerable element 1140 for steering the system 1100. One or both of the inner and outer members may further include a coating, as previously described.

## Claims

1. A navigation system (100, 800) comprising:
an inner member (110, 810),
an outer member (120, 830) having an inner diameter greater than the outer diameter of the inner member, the outer member comprising a flexible tube (600) with regions of different flexibility, a lumen between the inner and outer members, an alternative lumen through the outer member,
wherein the flexible tube (600) comprises a camera element; and
a fluid management system at a proximal end of outer member (830) comprising a fluid connector (740) having injection ports; a mixer (860); a timed infusion element (870); a flushing element (880) and a protective shield (864).

2. The navigation system of claim 1, wherein the inner member comprises a guidewire.

3. The navigation system of claim 2, wherein the system comprises a series of inner members (410, 420, 430) of varying diameters or stiffness.

4. The navigation system of claim 1, wherein one of the members further includes an ultrasound sensor (230).

5. The navigation system of claim 1, wherein one of the members further includes a pressure sensor (234).

6. The navigation system of claim 1, wherein one of the members further includes a tissue excision or ablation element (550).

7. The navigation system of claim 1, further including a tissue removal element (460).

8. The navigation system of claim 1, wherein one of the members comprises a coating (124).

9. The navigation system of claim 8, wherein the coating comprises PTFE, PET, a hydrophobic coating, a superhydrophobic coating, a hydrophilic coating, an anti-inflammatory coasting, an anti-bacterial coating, an anti-microbial coating, or an electrically responsive coating.

10. The navigation system of claim 1, further including a locking element (760) for fixing the inner member to the outer member(s).

11. The navigation system of claim 1, wherein one of the members is configured to deliver directed magnetic energy for directing drug delivery or an instrument to a target site.

12. The navigation system of claim 1 further comprising: one of the members having an imaging capability comprising ultrasound, magnetic imaging, or imaging modality in addition to fluoroscopic imaging.

13. The navigation system of claim 1 wherein one of the members includes a cutting wire (550).

14. The navigation system of claim 1 wherein one of the members includes a needle knife (1050) for dissecting and energizing tissue.

15. The navigation system of claim 1, wherein the system is remotely steerable through one or more of its members.

## Patentansprüche

1. Navigationssystem (100, 800), umfassend:
ein inneres Element (110, 810),
ein äußeres Element (120, 830), das einen Innendurchmesser, der größer ist als der Außendurchmesser des inneren Elements, aufweist, wobei das äußere Element einen flexiblen Schlauch (600) mit Bereichen unterschiedlicher Flexibilität, ein Lumen zwischen dem inneren und dem äußeren Element sowie ein alternatives Lumen durch das äußere Element umfasst,
wobei der flexible Schlauch (600) ein Kameraelement umfasst; und
ein Fluidmanagementsystem an einem proximalen Ende des äußeren Elements (830), das einen Fluidverbinder (740) umfasst, der Injektionsanschlüsse; einen Mischer (860); ein zeitgesteuertes Infusionselement (870); ein Spülelement (880) und eine Schutzabdeckung (864) aufweist.

2. Navigationssystem nach Anspruch 1, wobei das innere Element einen Führungsdraht umfasst.

3. Navigationssystem nach Anspruch 2, wobei das System eine Reihe von inneren Elementen (410, 420, 430) mit variierenden Durchmessern oder Steifigkeit umfasst.

4. Navigationssystem nach Anspruch 1, wobei eines der Elemente weiter einen Ultraschallsensor (230) einschließt.

5. Navigationssystem nach Anspruch 1, wobei eines der Elemente weiter einen Drucksensor (234) einschließt.

6. Navigationssystem nach Anspruch 1, wobei eines der Elemente weiter ein Gewebeexzisions- oder -ablationselement (550) einschließt.

7. Navigationssystem nach Anspruch 1, das weiter ein Gewebeentfernungselement (460) einschließt.

8. Navigationssystem nach Anspruch 1, wobei eines der Elemente eine Beschichtung (124) umfasst.

9. Navigationssystem nach Anspruch 8, wobei die Beschichtung PTFE, PET, eine hydrophobe Beschichtung, eine superhydrophobe Beschichtung, eine hydrophile Beschichtung, eine entzündungshemmende Beschichtung, eine antibakterielle Beschichtung, eine antimikrobielle Beschichtung oder eine elektrisch ansprechbare Beschichtung umfasst.

10. Navigationssystem nach Anspruch 1, das weiter ein Verriegelungselement (760) zur Befestigung des inneren Elements an dem/den äußeren Element(en) einschließt.

11. Navigationssystem nach Anspruch 1, wobei eines der Elemente so konfiguriert ist, dass es gerichtete magnetische Energie zum Leiten von Arzneimittelabgabe oder eines Instruments zu einer Zielstelle abgibt.

12. Navigationssystem nach Anspruch 1 weiter umfassend:
dass eines der Elemente eine Bildgebungsfähigkeit aufweist, die Ultraschall, Magnetresonanztomographie oder eine andere Bildgebungsmodalität zusätzlich zu Fluoroskopiebildgebung umfasst.

13. Navigationssystem nach Anspruch 1, wobei eines der Elemente einen Schneiddraht (550) einschließt.

14. Navigationssystem nach Anspruch 1, wobei eines der Elemente ein Nadelmesser (1050) zum Sezieren und Erregen von Gewebe einschließt.

15. Navigationssystem nach Anspruch 1, wobei das System über eines oder mehrere seiner Elemente fernsteuerbar ist.

## Revendications

1. Système de navigation (100, 800) comprenant :
un élément intérieur (110, 810),
un élément extérieur (120, 830) présentant un diamètre intérieur supérieur au diamètre extérieur de l'élément intérieur, l'élément extérieur comprenant un tube flexible (600) avec des régions de flexibilité différente, une lumière entre les éléments intérieur et extérieur, une lumière alternative à travers l'élément extérieur,
dans lequel le tube flexible (600) comprend un élément de caméra ; et
un système de gestion de fluide à une extrémité proximale de l'élément extérieur (830) comprenant un connecteur de fluide (740) présentant des ports d'injection ; un mélangeur (860) ; un élément d'infusion temporisé (870) ; un élément de rinçage (880) et un écran de protection (864).

2. Système de navigation selon la revendication 1, dans lequel l'élément intérieur comprend un fil-guide.

3. Système de navigation selon la revendication 2, dans lequel le système comprend une série d'éléments intérieurs (410, 420, 430) de diamètres ou de rigidités variables.

4. Système de navigation selon la revendication 1, dans lequel l'un des éléments inclut en outre un capteur à ultrasons (230).

5. Système de navigation selon la revendication 1, dans lequel l'un des éléments inclut en outre un capteur de pression (234).

6. Système de navigation selon la revendication 1, dans lequel l'un des éléments inclut en outre un élément d'excision ou d'ablation de tissu (550).

7. Système de navigation selon la revendication 1, incluant en outre un élément d'élimination de tissus (460).

8. Système de navigation selon la revendication 1, dans lequel l'un des éléments comprend un revêtement (124).

9. Système de navigation selon la revendication 8, dans lequel le revêtement comprend du PTFE, du PET, un revêtement hydrophobe, un revêtement superhydrophobe, un revêtement hydrophile, un revêtement anti-inflammatoire, un revêtement antibactérien, un revêtement antimicrobien ou un revêtement électriquement réactif.

10. Système de navigation selon la revendication 1, incluant en outre un élément de verrouillage (760) pour fixer l'élément intérieur à ou aux éléments extérieurs.

11. Système de navigation selon la revendication 1, dans lequel l'un des éléments est configuré pour fournir une énergie magnétique dirigée afin de diriger l'administration d'un médicament ou d'un instrument vers un site cible.

12. Système de navigation selon la revendication 1, comprenant en outre :
l'un des éléments présentant une capacité d'imagerie comprenant l'échographie, l'imagerie magnétique ou une modalité d'imagerie en plus de l'imagerie fluoroscopique.

13. Système de navigation selon la revendication 1, dans lequel l'un des éléments inclut un fil de coupe (550).

14. Système de navigation selon la revendication 1, dans lequel l'un des éléments inclut un couteau à aiguille (1050) pour disséquer et dynamiser les tissus.

15. Système de navigation selon la revendication 1, dans lequel le système est pilotable à distance par l'intermédiaire d'un ou plusieurs de ses éléments.
